(19) Europäisches Patentamt
European Patent Office
Office européen des brevets

(11) Veröffentlichungsnummer: **0 005 545**
**B1**

(12) **EUROPÄISCHE PATENTSCHRIFT**

(45) Veröffentlichungstag der Patentschrift: **18.09.85**

(21) Anmeldenummer: **79101509.2**

(22) Anmeldetag: **17.05.79**

(51) Int. Cl.$^4$: **C 07 D 233/84,**
C 07 D 405/04,
C 07 D 409/04,
C 07 D 401/04,
A 61 K 31/415, A 61 K 31/44

(54) **Neue Imidazolderivate, Verfahren zu ihrer Herstellung und diese enthaltende pharmazeutische Präparate.**

(30) Priorität: **24.05.78 DE 2823197**

(43) Veröffentlichungstag der Anmeldung:
**28.11.79 Patentblatt 79/24**

(45) Bekanntmachung des Hinweises auf die Patenterteilung:
**18.09.85 Patentblatt 85/38**

(84) Benannte Vertragsstaaten:
**AT BE CH DE FR GB IT LU NL SE**

(56) Entgegenhaltungen:
**EP-A-0 000 353**
**EP-A-0 004 648**
**EP-A-0 019 688**
**DE-A-2 338 279**
**DE-A-2 635 876**
**DE-A-2 805 166**
**US-A-2 981 739**
**US-A-3 714 179**

(73) Patentinhaber: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Niedballa, Ulrich, Dr.**
**Gosslerstrasse 28a**
**D-1000 Berlin 33 (DE)**
Erfinder: **Böttcher, Irmgard, Dr.**
**Rodelbahnpfad 5**
**D-1000 Berlin 28 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 005 545 B1

**Beschreibung**

Die Erfindung betrifft Imidazol-Derivate zur Behandlung entzündlicher oder allergischer Erkrankungen des Menschen, die gekennzeichnet sind durch die allgemeine Formel I

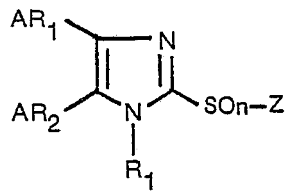

(I),

worin

n die Ziffern 0, 1 oder 2 darstellt, worin $AR_1$ und $AR_2$ einen durch Halogenatome, Alkylreste mit 1 bis 4 Kohlenstoffatomen oder Alkoxyresten mit 1 bis 4 Kohlenstoffatomen substituierter Phenylrest bedeutet, worin $R_1$ ein Wasserstoffatom oder eine Alkylgruppe mit 1 bis 4 Kohlenstoffatomen darstellt und worin

Z eine durch ein oder zwei Hydroxygruppen, Alkoxygruppen mit 1 bis 4 Kohlenstoffatomen, oder Alkylendioxygruppen mit 2 bis 6 Kohlenstoffatomen substituierter Alkylrest mit 2 bis 6 Kohlenstoffatomen bedeutet und deren Salze mit physiologisch unbedenklichen Säuren.

Erfindungsgemäß bedeuten die Substituenten $AR_1$ und $AR_2$ der Imidazolderivate jeweils einen durch Halogenatome, Alkylgruppen oder Alkoxygruppen substituierten Phenylrest.

Durch Halogenatome substituierte Phenylreste $AR_1$ und $AR_2$ sind beispeilsweise Mono- oder Difluorphenylreste oder Mono- oder Dichlorphenylreste und insbesondere para-Fluorphenylreste oder para-Chlorphenylreste. Alkylsubstituierte Phenylreste sind solche, deren Alkylgruppen 1 bis 4 Kohlenstoffatome (beispielsweise Methyl-, Äthyl-, Propyl- oder Isopropylgruppen) enthalten. Durch Alkoxygruppen substituierte Phenylreste sind solche, deren Alkoxygruppen 1 bis 4 Kohlenstoffatome (Methoxy-, Äthoxy-, Propyloxy- oder Isopropyloxygruppen enthalten.

Die Substituenten $AR_1$ und $AR_2$ können gleich oder verschieden sein.

Erfindungsgemäß bedeutet der Substituent $R_1$ ein Wasserstoffatom oder einen Alkylrest mit 1 bis 4-Kohlenstoffatome — insbesondere einen Methylrest.

Z bedeutet eine durch ein oder zwei Hydroxygruppen, Alkoxygruppen mit 1 bis 4 Kohlenstoffatomen, oder Alkylendioxygruppen mit 2 bis 6 Kohlenstoffatomen substituierter Alkylrest mit 2 bis 6 Kohlenstoffatomen. Die Alkoxygruppen des Substituenten Z sind beispielsweise Methoxygruppen, Ethoxygruppen, Propyloxygruppen oder Isopropyloxygruppen. Alkylendioxygruppen des Substituenten Z sind beispielsweise die 1,2-Ethylendioxygruppen, der 1,3-Propylendioxygruppe oder die 2,2-Dimethylpropylendioxygruppe.

Die Erfindung betrifft insbesondere solche Imidazol-Derivate der allgemeinen Formel I, in denen der Substituent Z eine Hydroxyalkylgruppe mit 2 bis 6 Kohlenstoffatomen — insbesondere eine 2-Hydroxyäthylgruppe-, eine 1,3-Dioxolan-2-yl-methylgruppe oder eine 2,2-Diäthoxyäthylgruppe bedeutet.

Physiologisch unbedenkliche Salze der Imidazol-Derivate der allgemeinen Formel I sind beispeilsweise Salze des Chlorwasserstoffs, Bromwasserstoffs oder Jodwasserstoffs, der Schwefelsäure, Phosphorsäure oder Salze organischer Säuren wie der Ameisensäure, Essigsäure, Bernsteinsäure, Maleinsäure, Weinsäure oder Zitronensäure.

Der erfindungsgemäßen Verbindungen zeichnen sich gegenüber den aus der DE—A 26 35 876 vorbekannten Verbindungen, wie an späterer Stelle dargelegt wird, dadurch aus, daß sie bei etwa gleicher Wirksamkeit signifikant weniger Magenulcera erzeugen. Von den Verbindungen der EP—A 79 100 962, die gemäß Art. 54 (3) EPÜ Stand der Technik ist, unterscheiden sich die erfindungsgemäßen Verbindungen dadurch, daß beide Substituenten $AR_1$ und $AR_2$ gegebenenfalls substituierte Phenylreste bedeuten. Ebenfalls zum Stand der Technik gemäß Art. 54 (3) sind die EP—A 78 100 272 und 80 101 323 zu rechnen, in denen einige Thioverbindungen der allgemeinen Formel I als Zwischenprodukte vorbeschrieben sind.

Eine besonders ausgeprägte antiinflammatorische und antiallergische Wirksamkeit besitzen die Imidazol-Derivate der allgemeinen Formel I, in denen die Substituenten $AR_1$ und $AR_2$ einen 4-Fluorphenylrest, einen 4-Chlorphenylrest, einen 4-Methylphenylrest oder 4-Methoxyphenylrest bedeuten und in denen der Substituent Z eine Hydroxyalkylgruppe mit 2 bis 6 Kohlenstoffatomen — insbesondere eine 2-Hydroxyäthylgruppe —, eine 1,3-Dioxolan-2-yl-methylgruppe oder eine 2,2-Diäthoxyäthylgruppe darstellt.

Eine besonders günstige Dissoziation zwischen erwünschter pharmakologischer Wirksamkeit und unerwünschter — insbesondere ulcerogener — Nebenwirkung haben die Imidazol-Derivate der allgemeinen Formel I, in denen n die Bedeutung der Ziffern 1 oder 2 hat.

Die antiinflammatorische Wirksamkeit der erfindungsgemäßen Substanzen kann mit Hilfe des bekannten Adjuvans-Arthritis-Testes ermittelt werden, der wie folgt durchgeführt wird:

Es werden weibliche und männliche Ratten des Stammes Lewis (LEW) in der Gewichtsspanne zwischen 110—190 g verwendet. Die Tiere erhalten Trinkwasser und Altromin-Preßfutter ad libitum.

Für jede Dosisgruppe werden 10 Ratten eingesetzt.

Mycobacterium butyricum der Firma Difko, Detroit wird als Reizmittel verwandt. Eine Suspension von 0,5 mg Mycobacterium butyricum in 0,1 ml dünnflüssigem Paraffin (DAB 7) wird in die rechte Hinterpfote subplantar injiziert.

Die Testsubstanzen werden vom 11. Versuchstag an täglich über 4 Tage oral gegeben. Die Substanzen werden als klare wässrige Lösung oder als Kristallsuspension unter Zusatz von Myrj 53 (85 mg %) in isotonischer Natriumchlorid-Lösung verabreicht.

Versuchsansatz:

Die Rattel werden in bezug auf ihr Körpergewicht möglichst gleichmäßig in verschiedene Gruppen eingeteilt. Nach plethysmographischer Volumenmessung der rechten Hinterpfote wird in diese subplantar 0,1 ml Adjuvans injiziert. Die rechten Hinterpfoten werden vom 14. Versuchstag bis zum Versuchsende gemessen. Die Versuchsdauer beträgt 3 Wochen.

Bestimmt wird die Abheilung der Hinterpfoten, welche bei der vorgegebenen Dosis erzielt wird.

Eine häufige Komplikation bei der Therapie mit nichtsteroidalen Entzündungshemmern stellt das Auftreten von Magenulcerationen dar. Diese Nebenwirkung kann im Tierversuch nachgewiesen werden, wobei bei vorgebener Dosis die Anzahl der beobachteten Läsionen und deren Gesamtfläche ermittelt wird. Der Ulkus-Test wird die folgt durchgeführt.

Es werden männliche Wistar-Ratten (SPF) verwandt. Die Tiere liegen in einer Gewichtsspanne von 130 ± 10 g. 16 Stunden vor Versuchsbeginn werden die Tiere vom Futter abgesetzt; sie erhalten Wasser ad libitum.

Pro Dosis werden 5 Tiere eingesetzt. Die Substanzen werden einmal oral, in Natriumchlorid gelöst oder als Kristallsuspension unter Zusatz von 85 mg % Myri 53 appliziert.

3 Stunden nach Substanzapplikation injiziert man 1 ml einer 3 %igen Lösung des Farbstoffs Diphenylreinblau intravenös und tötet das Tier. Der Magen wird reseziert und mikroskopisch auf Anzahl und Gesamtgröße von Epithelläsionen und Ulcera, die durch Farbstoffanreicherungen hervortreten, untersucht.

Es wird der Faktor ermittelt um den sich die Lasionen nach Anzahl und Fläche gegenüber den Lasionen der entsprechend behandelten Kontrolltiere vervielfacht hat.

Die nachfolgende Tabelle zeigt die in diesen Testen erhaltenen Ergebnisse der erfindungsgemäßen Verbindungen im Vergleich zum vorbekannten Indomethazin (Substanz 1) und zu den aus der deutschen Offenlegungsschrift 26 35 876 vorbekannten strukturanalogen Verbindungen 2 und 3.

Die Ergebnisse, daß die erfindungsgemäßen Verbindungen bei gleicher Wirksamkeit signifikant weniger Magenulcera verursachen als die aus der obigen Offenlegungsschrift vorbekannten strukturanalogen Substanzen.

TABELLE

| Nr. | Substanz | Adjuvans-Arthritis-Test | | Ulcus-Test | | |
|---|---|---|---|---|---|---|
| | | Dosis in mg/kg Tier | % Abheilung der rechten Pfoten | Dosis in mg/kg Tier | Faktor für die Anzahl | Faktor für die Fläche |
| 1 | Indomethacin | 4 × 4 mg | 48—58% | 8 mg | über 20 | über 30 |
| 2 | 4,5-Bis-(4-methoxyphenyl)-2-äthylthio-imidazol (bekannt) | 4 × 50 mg | 46% | 200 mg | 13,5 | 30 |
| 3 | 4,5-Bis-(4-methoxyphenyl)-2-äthylsulfonyl-imidazol (bekannt) | 4 × 50 mg | 53% | 200 mg | 4,5 | 4,5 |
| 4 | 4,5-Bis-(4-methoxyphenyl)-2-(2-hydroxyäthylthio)-imidazol | 4 × 50 mg | 45% | 200 mg | 4,4 | 8,7 |
| 5 | 4,5-Bis-(4-methoxyphenyl)-2-(2-hydroxyäthylsulfinyl)-imidazol | 4 × 50 mg | 52% | 200 mg | 1,0 | 1,0 |
| 6 | 4,5-Bis-(4-methoxyphenyl)-2-(2-Hydroxyäthylsulfonyl)-imidazol | 4 × 50 mg | 51% | 200 mg | 1,2 | 1,2 |
| 7 | 4,5-Bis-(4-chlorphenyl)-2-(2-hydroxyäthylsulfonyl)-imidazol | 4 × 50 mg | 49% | 200 mg | 0,5 | 0,5 |
| 8 | 4,5-Bis-(4-methoxyphenyl)-2-(2,2-dimethoxyäthylsulfonyl)-imidazol | 4 × 50 mg | 49% | 200 mg | 2,6 | 2,3 |
| 9 | [4,5-Bis-(4-methoxyphenyl)-2-imidazolyl)]-(1,3-dioxolan-2-yl-methyl)-sulfon | 4 × 50 mg | 45% | 200 mg | 1,8 | 1,8 |

Überraschenderweise gibt es unter den erfindungsgemäßen Verbindungen auch solche, die zusätzlich zur antiinflammatorischen Wirksamkeit noch eine ausgeprägte antiulcerogene und tumorhemmende Wirksamkeit besitzen.

So zeigen Ratten die neben 8 mg/kg Körpergewicht Indomethacin zusätzlich noch 50 mg/kg Körpergewicht 4,5-Bis-(4-methoxyphenyl)-2-(2-hydroxyäthylsulfinyl)-imidazol erhalten, nach Anzahl und Fläche signifikant weniger Magenlasionen als eine entsprechende Kontrollgruppe der nur Indomethacin appliziert wird.

Andererseits vermag eine Dosis von 50 mg/kg Tier Bis(4-methoxyphenyl)-2-(2-hydroxyäthylsulfinyl)-imidazol die Tumorbildung von mit 100 000 Ehrlich-Tumorzellen infizierten Ratten signifikant zu unterdrücken.

Somit eignen sich die neuen Verbindungen in Kombination mit den in der galenischen Pharmazie üblichen Trägermitteln zur Behandlung zum Beispiel von akuter und chronischer Polyarthritis, Neurodermitis, Asthma bronchiale und Heufieber.

Die Herstellung der Arzneimittelspezialitäten erfolgt in üblicher Weise, indem man die Wirkstoffe mit geeigneten Zusätzen, Trägersubstanzen und Geschmackskorriegentien in die gewünschten Applikationsformen wie Tabletten, Dragees, Kapseln, Lösungen und Inhalationsmitteln überführt.

Für die orale Anwendung eignen sich insbesondere Tabletten, Dragees und Kapseln, welche beispielsweise 1 bis 250 mg Wirkstoff und 50 mg bis 2 g pharmakologisch unwirksamen Trägers, wie zum Beispiel Laktose, Amylose, Talkum, Gelatine, und Magnesiumstearat, sowie mit üblichen Zusätze enthalten.

Die neuen Imidazol-Derivate der allgemeinen Formel I gemäß Anspruch 1 können nach an sich bekannten Verfahren hergestellt werden. Ein geeignetes Herstellungsverfahren ist beispielsweise dadurch gekennzeichnet, daß man in an sich bekannter Weise

a) ein Imidazol-Derivat der allgemeinen Formel II

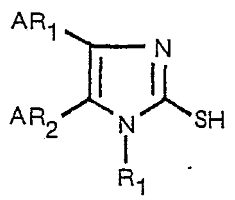

(II),

worin $AR_1$, $AR_2$ und $R_1$ die obengenannte Bedeutung besitzt, mit einer Verbindung der allgemeinen Formel III

$$W—Z \qquad (III),$$

worin Z die obengenannter Bedeutung besitzt und worin W ein Halogenatom, einen Alkylsulfonyloxyrest oder einen Arylsulfonyloxyrest darstellt, kondensiert, oder

b) zur Herstellung von Imidazol-Derivaten der allgemeinen Formel I mit Z in der Bedeutung einer Gruppierung

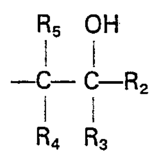

worin $R_2$, $R_3$, $R_4$ und $R_5$ Wasserstoffatome oder gegebenenfalls durch eine Hydroxygruppe, Alkoxygruppe oder Alkylendioxygruppe substituierte Kohlenwasserstoffreste mit insgesamt 1 bis 4 Kohlenstoffatomen bedeuten, an ein Imidazol-Derivat der allgemeinen Formel II

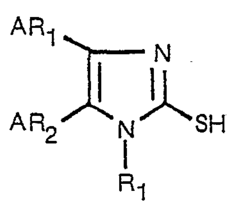

(II),

worin $AR_1$, $AR_2$ und $R_1$ die obengenannte Bedeutung besitzen, ein Epoxid der allgemeinen Formel IV

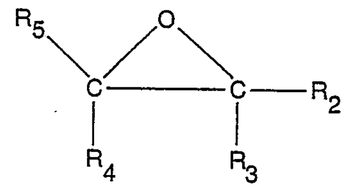

worin $R_2$, $R_3$, $R_4$ und $R_5$ die obengenannte Bedeutung besitzen addiert und gegebenenfalls die gemäß Verfahrensvariante a oder b erhaltenen Thioverbindungen der allgemeinen Formel I zu den entsprechenden Sulfinylverbindungen oder Sulfonylverbindungen oxydiert, gegebenenfalls in 1-Stellung unsubstituierte Imidazol-Derivate alkyliert und/oder Imidazol-Derivate der allgemeinen Formel I mit physiologisch unbedenklichen Säuren in ihre Salze überführt.

Dieses Verfahren kann unter den bekannten Bedingungen durchgeführt werden (Liebigs Annalen *284,* 1894, Seite 9, f., J. Chem. Soc. 1931, Seite 3043 f., J. Med. Chem. *20,* 1977, Seite 563 f., Liebigs Annalen *214,* 1882, Seite 257, f., J. Chem. Soc., 1942, Seite 232 f., J. Chem. Soc. 1963, Seite 2195 f., Houben Weyl: Methoden der organischen Chemie Band IX, Seite 229 f., Bull Soc. France 1977, Seite 271 f., Deutsche Offenlegungsschrift 26 35 876).

Nach erfolgter Synthese können die racemischen Imidazol-Derivate der allgemeinen Formel I gemäß Anspruch 1 in an sich bekannter Weise in ihre optischen Antipoden gespalten werden, beispielsweise indem man diese durch Säulenchromatographie an optisch aktiven Trägern (z.B. Sephadex(R)) chromatographiert.

Die Ausgangsverbindungen für das erfindungsgemäße Verfahren sind bekannt oder können in an sich bekannter Weise hergestellt werden (Synthesis 1976, Seite 733 f. und Zhur, Obsch. Khim 31, 1961, Seite 1039 f.).

Die nachfolgenden Beispiele dienen zur Erläuterung des erfindungsgemäßen Verfahrens.

Beispiel 1

Ein Gemisch von 6,24 g 4.5-Bis-(4-methoxy-phenyl)-2-mercaptoimidazol und 3,0 g 2-Bromäthanol in 100 ml abs. Äthanol wird 4 Stunden unter Feuchtigkeitsausschluß und Argonabdeckung am Rückfluß gekocht. Die auf 5° abgekühlte Lösung wird durch Zugabe von 2 n Natronlauge neutralisiert, in 900 ml Eiswasser gegossen und bei Eisbadtemperatur bis zur Beendigung der Kristallisation stehengelassen. Nach 45 Minuten werden die Kristalle abgesaugt, mit Eiswasser gewaschen und bei 70° im Vakuum getrocknet. Das Rohprodukt wird aus 250 ml Essigester unter Zusatz von wenig Aktivkohle umkristallisiert, und man erhält 5,38 g 4.5-Bis-(4-methoxyphenyl)-2-(2-hydroxyäthylthio)imidazol als farblose Kristalle vom Schmelzpunkt 182°C.

Beispiel 2

Zu der Lösung von 5,34 g 4.5-Bis-(4-methoxyphenyl)-2-(2-hydroxy-äthylthio)-imidazol in 1,25 l Dichlormethan werden 3,30 g 3-Chlorperbenzoesäure, in 150 ml Dichlormethan gelöst, getropft. Die Lösung wird 24 Stunden bei Raumtemperatur gerührt und dann mit gesättigter Natriumhydrogenkarbonatlösung gewaschen. Die organische Lösung wird abgetrennt und über Natriumsulfat getrocknet und im Vakuum eingeengt. Der Rückstand wird aus 30 ml Dioxan unter Zusatz von wenig Aktivkohle kristallisiert, und man erhält 3,01 g 4.5-Bis-(4-methoxyphenyl)-2-(2-hydroxyäthylsulfinyl)-imidazol vom Schmelzpunkt 184°C.

Beispiel 3

Zu einer Lösung von 2,14 g 4,5-Bis(4-methoxyphenyl)-2-(2-hydroxy-äthylthio)-imidazol in 450 ml Dichlormethan werden 2,70 g 3-Chlorperbenzoesäure, in 120 ml Dichlormethan gelöst, getropft. Man rührt 4 Stunden bei Raumtemperatur, wäscht mit gesättigter Natriumhydrogenkarbonatlösung, trennt die organische Phase ab, trocknet sie über Natriumsulfat und engt sie im Vakuum ein. Der ölige Rückstand wird aus 150 ml Cyclohexan/Äther 1:1 umkristallisiert, und man erhält 1,68 g 4.5-Bis-(4-methoxyphenyl)-2-(2-hydroxyäthylsulfonyl)-imidazol vom Schmelzpunkt 168°C.

Beispiel 4

Zu einer Lösung von 7,13 g 4.5-Bis-(4-methoxyphenyl)-2-(2-hydroxyäthylthio)-imidazol in 100 ml absolutem Äthanol, in dem 600 mg Natrium gelöst waren, werden 3,73 g Methyljodid in 20 ml abs. Äthanol gegeben. Die Lösung wird 20 Minuten unter Argon am Rückfluß erhitzt. Nach dem Abkühlen wird in 1500 ml Eiswasser gegossen, mit 2 n Salzsäure neutralisiert und mit Chloroform extrahiert. Die organische Phase wird über Natriumsulfat getrocknet und im Vakuum eingeengt. Das Rohprodukt wird aus 100 ml Äthanol unter Zusatz von wenig Aktivkohle und 1000 ml Diisopropyläther umkristallisiert, und man erhält 4,29 g 4.5-Bis-(4-methoxyphenyl)-2-(2-hydroxyäthylthio)-1-methyl-imidazol vom Schmelzpunkt 127°C.

Beispiel 5

Ein Gemisch von 8,65 g 4.5-Bis-(4-fluorphenyl)-2-mercaptoimidazol und 4,97 g 2-Bromäthanol in 150 absolutem Äthanol wird 4 Stunden lang unter Argon am Rückfluß erhitzt. Die abgekühlte Lösung wird mit 2

n Natronlauge neutralisiert, in 800 ml Eiswasser gegossen und 45 Minuten lang bei Eisbadtemperatur belassen. Man saugt das Rohprodukt ab, wäscht es mit Wasser und trocknet es im Vakuum bei 70°. Das Rohprodukt wird aus 175 ml Essigester unter Zusatz von wenig Aktivkohle umkristallisiert, und man erhält 6,76 g 4.5-Bis-(4-fluorphenyl)-2-(2-hydroxyäthylthio)-imidazol vom Schmelzpunkt 192°C.

Beispiel 6

Unter den Bedingungen des Beispiels 2 werden 0,5 g 4.5-Bis-(4-fluorphenyl)-2-(2-hydroxyäthylthio)-imidazol oxydiert, und man erhält 0,24 g 4.5-Bis-(4-fluorphenyl)-2-(2-hydroxyäthylsulfinyl)imidazol vom Schmelzpunkt 182°C.

Beispiel 7

Unter den Bedingungen des Beispiels 3 werden 0,6 g 4.5-Bis-(4-fluorphenyl)-2-(2-hydroxyäthylthio)-imidazol oxydiert, und man erhält 4.5-Bis-(4-fluorphenyl)-2-(2-hydroxyäthylsulfonyl)imidazol vom Schmelzpunkt 167°C.

Beispiel 8

Ein Gemisch aus 8,03 g 4.5-Bis-(4-chlorphenyl)-2-mercaptoimidazol und 4,14 g 2-Bromäthanol in 125 ml absolutem Äthanol wird 4 Stunden lang unter Argon am Rückfluß erhitzt. Die abgekühlte Lösung wird mit 2 n Natronlauge neutralisiert, in 1200 ml Eiswasser gegossen und 45 Minuten lang bei Eisbadtemperatur belassen. Man saugt die Kristalle ab, wäscht sie mit Wasser und trocknet sie im Vakuum bei 60°. Das Rohprodukt wird aus 500 ml Acetonitril unter Zusatz von wenig Aktivkohle umkristallisiert, und man erhält 8,76 g 4.5-Bis-(4-chlorphenyl)-2-(2-hydroxyäthylthio)imidazol vom Schmelzpunkt 202°C.

Beispiel 9

Unter den Bedingungen des Beispiels 2 werden 0,5 g 4.5-Bis-(4-chlorphenyl)-2-(2-hydroxyäthylthio)-imidazol oxydiert, und man erhält 0,38 g 4.5-Bis-(4-chlorphenyl)-2-(2-hydroxyäthylsulfinyl)-imidazol vom Schmelzpunkt 186°C.

Beispiel 10

Unter dem Bedingungen des Beispiels 3 werden 0,5 g 4.5-Bis-(4-chlorphenyl)-2-(2-hydroxyäthylthio)-imidazol oxydiert, und man erhält 0,41 g 4.5-Bis-(4-chlorphenyl)-2-(2-hydroxyäthylsulfonyl)-imidazol vom Schmelzpunkt 191°C.

Beispiel 11

Ein Gemisch aus 4,20 g 4.5-Bis-(p-tolyl)-2-mercapto-imidazol und 2,49 g 2-Bromäthanol in 75 ml absolutem Äthanol wird 4,5 Stunden unter Argon am Rückfluß erhitzt. Die auf 5°C abgekühlte Lösung wird durch Zugabe von 2 n Natronlauge neutralisiert, in 800 ml Eiswasser gegossen und 45 Minuten bei Eisbadtemperatur belassen. Es erfolgte Kristallisation. Dann saugt man die Kristalle ab, wäscht sie mit Wasser und trocknet sie im Vakuum bei 60°C. Das Rohprodukt wird aus 200 ml Acetonitril unter Zusatz von wenig Aktivkohle umkristallisiert, und man erhält 3,95 g 4.5-Bis-(p-tolyl)-2-(2-hydroxyäthylthio)-imidazol vom Schmelzpunkt 182°C.

Beispiel 12

Unter den Bedingungen des Beispiels 2 werden 0,5 g 4.5-Bis-(p-tolyl)-2-(2-hydroxyäthylthio)-imidazol oxydiert, und man erhält 0,33 g 4.5-Bis-(p-tolyl)-2-(2-hydroxyäthylsulfinyl)-imidazol vom Schmelzpunkt 169°C.

Beispiel 13

Unter den Bedingungen des Beispiels 3 werden 0,6 g 4.5-Bis-(p-tolyl)-2-(2-hydroxyäthylthio)-imidazol oxydiert, und man erhält 0,50 g 4.5-Bis-(p-tolyl)-2-(2-hydroxyäthylsulfonyl)-imidazol vom Schmelzpunkt 166°C.

Beispiel 14

Ein Gemisch aus 6,25 g 4.5-Bis-(4-methoxy-phenyl)-2-mercaptoimidazol und 3,66 g 2-Brompropionsäureäthylester in 100 ml absolutem Äthanol wird 2,5 Stunden lang unter Argon am Rückfluß erhitzt. Die abgekühlte Lösung wird mit 2 n Natronlauge neutralisiert und in 1000 ml Eiswasser gegossen. Das ausgefallene Öl wird in Essigester aufgenommen, die Lösung über Natriumsulfat getrocknet und im Vakuum eingeengt. Man erhält 7,12 g [4.5-Bis-(4-methoxyphenyl)-2-imidazolyl]-2-thiopropionsäureäthylester als farbloses Öl.

Elementaranalyse:

| | | | | | |
|---|---|---|---|---|---|
| $C_{22}H_{24}N_2O_4S$ (412.52) | Ber. | C 66.64 | H 6.10 | N 7.06 | S 8.09% |
| | Gef. | 66.41 | 6.23 | 7.15 | 8.21% |

Beispiel 15

Zu einer Lösung von 8,25 g [4.5-Bis-(4-methoxyphenyl)-2-imidazolyl]-2-thio-propionsäureäthylester in 150 ml absolutem Tetrahydrofuran werden anteilweise 0,607 g Lithiumaluminiumhydrid gegeben. Es wird noch 30 Minuten bei Raumtemperatur gerührt, mit gesättigter Ammoniumchloridlösung zersetzt und mit Essigester extrahiert. Nach Trocknen der organischen Lösung über Natriumsulfat wird im Vakuum zur Trockne eingeengt. Der kristalline Rückstand wird aus Toluol umkristallisiert, und man erhält 5,07 g [4.5-Bis-(4-methoxyphenyl)]-2-(2-hydroxy-1-methyl-äthylthio)-imidazol vom Schmelzpunkt 141°C.

Beispiel 16

Zu einer Lösung von 3,71 g [4.5-Bis-(4-methoxyphenyl]-2-(2-hydroxy-1-methyläthylthio)-imidazol in 200 ml Chloroform wird bei Raumtemperatur eine Lösung von 2,16 g 3-Chlorperbenzoesäure in 150 ml Chloroform getropft. Es wird über Nacht bei Raumtemperatur gerührt und mit gesättigter Natriumhydrogenkarbonatlösung gewaschen. Man trocknet die organische Lösung über Natriumsulfat und engt sie im Vakuum ein. Der Rückstand wird durch Chromatographie an 200 g Kieselgel mit Essigester als Elutionsmittelk gereinigt. Man erhält nach Abziehen des Lösungsmittels 2,67 g [4.5-Bis-(4-methoxyphenyl)]-2-(2-hydroxy-1-methyl-äthylsulfinyl)-imidazol als amorphen Schaum.

| | | | | | |
|---|---|---|---|---|---|
| $C_{20}H_{22}N_2O_4S$ (386.47) | Ber. | C 62.16 | H 5,74 | N 7,25 | S 8,30 |
| | Gef. | 62,40 | 5,82 | 7,19 | 8,22 |

Beispiel 17

Zu einer Lösung von 3,71 g [4.5-Bis-(4-methoxyphenyl)]-2-(2-hydroxy-1-methyl-äthylthio)-imidazol in 200 ml Chloroform wird bei Raumtemperatur eine Lösung von 5,20 g 3-Chlorperbenzoesäure in 250 ml Chloroform getropft. Man rührt über Nacht bei Raumtemperatur, wäscht dann mit gesättigter Natriumbikarbonat-Lösung, trocknet, die organische Lösung über Natriumsulfat und engt sie im Vakuum zur Trockne eine. Man erhält 3,70 g [4.5-Bis-(4-methoxyphenyl)]-2-(2-hydroxy-1-methyl-äthylsulfonyl)-imidazol als amorphen Schaum vom Schmelzpunkt 68°C.

| | | | | | |
|---|---|---|---|---|---|
| $C_{20}H_{22}N_2O_5S$ (402,47) | Ber. | C 59,69 | H 5,51 | N 6,96 | S 7,97 |
| | Gef. | 59,51 | 5,61 | 7,08 | 7,88 |

Beispiel 18

Ein Gemisch aus 9,36 g 4.5-Bis-(4-methoxyphenyl)-2-mercaptoimidazol und 9,13 g 2-Bromisobuttersäureäthylester in 150 ml Äthanol wird 6 Stunden unter Argon am Rückfluß erhitzt. Die abgekühlte Lösung wird mit 2 n Natronlauge neutralisiert, in 600 ml Eiswasser gegossen, und man extrahiert das Rohprodukt mit Dichlormethan. Die organische Lösung wird über Natriumsulfat getrocknet und im Vakuum zur Trockne eingeengt. Der Rückstand kristallisiert aus Dichlormethan/Diäthyläther, und man erhält 11,29 g [4.5-Bis-(4-methoxyphenyl)-2-imidazolyl]-2-methyl-2-thiopropionsäureäthylester vom Schmelzpunkt 115°C.

Beispiel 19

8,53 g [4.5-Bis-(4-methoxyphenyl)-2-imidazolyl]-2-methyl-2-thiopropionsäureäthylester werden in einem Gemisch aus je 75 ml absolutem Tetrahydrofuran und Diäthyläther gelöst und anteilweise mit insgesamt 570 mg Lithiumaluminiumhydrid versetzt. Es wird 30 Minuten bei Raumtemperatur nachgerührt, mit gesättigter Ammonchloridlösung zersetzt und mit Essigester extrahiert. Nach Trocknen der organischen Lösung über Natriumsulfat wird im Vakuum zur Trockne eingeengt. Aus Toluol kristallisieren 6,33 g 4.5-Bis-(4-methoxyphenyl)-2-(1.1-dimethyl-2-hydroxyäthylthio)-imidazol vom Schmelzpunkt 195°C.

Beispiel 20

Unter den Bedingungen des Beispiels 2 werden 3,81 g 4.5-Bis-(4-methoxyphenyl)-2-(1.1-dimethyl-2-hydroxyäthylthio)-imidazol oxydiert, und man erhält 2,67 g 4.5-Bis-(4-methoxyphenyl)-2-(1.1-dimethyl-2-hydroxyathylsulfinyl)-imidazol vom Schmelzpunkt 215°C.

Beispiel 21

Unter den Bedingungen des Beispiels 3 werden 3,81 g 4.5-Bis-(4-methoxyphenyl)-2-(1.1-dimethyl-2-hydroxyäthylthio)-imidazol oxydiert, und man erhält 3,22 g 4.5-Bis-(4-methoxyphenyl)-2-(1.1-dimethyl-2-hydroxyäthylsulfonyl)-imidazol vom Schmelzpunkt 187°C.

Beispiel 22

Zu einer Lösung von 3,95 g 4.5-Bis-(4-methoxyphenyl)-2-mercaptoimidazol in 100 ml absolutem Methanol, in dem 320 mg Natrium gelöst sind, werden 2,14 g 2-Brombutyrolacton in 30 ml absolutem Methanol gegeben, und es wird 2,5 Stunden unter Argon am Rückfluß erhitzt. Nach dem Abkühlen gießt man die Lösung in 500 ml Eiswasser und neutralisiert mit 2 n Schwefelsäure. Man extrahiert das Produkt mit Essigester, trocknet die organische Lösung über Natriumsulfat und engt sie im Vakuum ein. Das verbleibende Öl wird aus Tetrahydrofuran/Hexan zur Kristallisation gebracht. Man erhält so 1,23 g [4.5-Bis-(4-methoxyphenyl)-2-imidazolyl]-2-thiobutyrolacton vom Schmelzpunkt 140—42°C.

Beispiel 23

Eine Lösung von 3,96 g [4.5-Bis-(4-methoxyphenyl)-2-imidazolyl]-2-thiobutyrolacton in 100 ml absolutem Tetrahydrofuran wird mit 300 mg Lithiumaluminiumhydrid versetzt. Es wird 30 Minuten bei Raumtemperatur nachgerührt, mit gesättigter Ammonchloridlösung zersetzt und mit Essigester extrahiert. Nach Trocken der organischen Lösung über Natriumsulfat wird im Vakuum zur Trockne eingeengt, und man erhält 3,26 g [4.5-Bis-(4-methoxyphenyl)-2-imidazolyl]-2-thio-1,4-butandiol in Form eines viskosen Öls.

| | | | | | |
|---|---|---|---|---|---|
| $C_{21}H_{24}N_2O_4S$ (400.50) | Ber. | C 62,98 | H 6,04 | N 7,00 | S 8,01 |
| | Gef. | 62,71 | 6,13 | 6,89 | 8,17 |

Beispiel 24

Zu einer Lösung von 6,25 g 4.5-Bis-(4-methoxyphenyl)-2-mercaptoimidazol in 50 ml absolutem Dimethylformamid werden 0,96 g 50% Natriumhydrid gegeben, und es wird 30 Minuten bei 60°C nachgerührt. Dann gibt man 2,74 g Chloracetaldehyddimethylacetal in 20 ml absolutem Dimethylformamid zu und rührt 4 Stunden unter Argon bei 80°C. Die abgekühlte Lösung wird in 700 ml Wasser gegossen und mit Chloroform extrahiert. Man trocknet die organische Lösung über Natriumsulfat, engt sie im Vakuum zur Trockne ein und reinigt den dunkelbraunen Rückstand durch Chromatographie an 500 g Kieselgel. Mit Essigester/Hexan im Verhältnis 1:3 eluiert man 5,01 g 4.5-Bis-(4-methoxyphenyl)-2-(2.2-dimethoxyäthylthio)-imidazol als hellgelbes Öl.

| | | | | | |
|---|---|---|---|---|---|
| $C_{21}H_{24}N_2O_4S$ (400.50) | Ber. | C 62,98 | H 6,04 | N 7,00 | S 8,01 |
| | Gef. | 62,69 | 6,16 | 7,08 | 8,11 |

Beispiel 25

Zu einer Lösung von 2,0 g 4.5-Bis-(4-methoxyphenyl)-2-(2.2-dimethoxyäthylthio)-imidazol in 150 ml Dichlormethan werden 1,19 g 3-Chlorperbenzoesäure, in 100 ml Dichlormethan gelöst, getropft, und es wird 4 Stunden bei Raumtemperatur gerührt. Dann wäscht man mit Natriumbikarbonat-Lösung, trocknet die organische Lösung über Natriumsulfat und engt sie im Vakuum zur Trockne ein. Der Rückstand wird aus Essigester/Hexan kristallisiert. Nach Umkristallisation aus Essigester/Äthanol erhält man 1,52 g 4.5-Bis-(4-methoxyphenyl)-2-(2.2-dimethoxyäthylsulfinyl)-imidazol vom Schmelzpunkt 127—28°C.

Beispiel 26

Zu einer Lösung von 2,5 g 4.5-Bis(4-methoxyphenyl)-2-(2.2-dimethoxyäthylthio)-imidazol in 150 ml Dichlormethan wird die Lösung von 2,97 g 3-Chlorperbenzoesäure in 100 ml Dichlormethan getropft, und es wird über Nacht bei Raumtemperatur gerührt. Dann wäscht man mit Natriumbikarbonat-Lösung, trocknet die organische Lösung über Natriumsulfat und engt sie im Vakuum zur Trockne ein. Der Rückstand wird aus Essigester/Hexan kristallisiert. Nach Umkristallisation aus Äthanol/Äther erhält man 1,96 g 4.5-Bis-(4-methoxyphenyl)-2-(2.2-dimethoxyäthylsulfonyl)-imidazol vom Schmelzpunkt 78—80°C.

Beispiel 27

Zu einer Lösung von 12,48 g 4.5-Bis-(4-methoxyphenyl)-2-mercaptoimidazol in 150 ml absolutem Dimethylformamid werden 1,92 g 50% Natriumhydrid gegeben. Nach 30 Minuten Rühren bei 60°C versetzt man mit 7,35 g Bromacetaldehydäthylenketal in 40 ml absolutem Dimethylformamid und rührt 4 Stunden bei 90°C unter Argon. Die erkaltete Lösung gießt man in 1000 ml Wasser, nimmt das ausgeschiedene Öl in Essigester auf, trocknet die organische Lösung über Natriumsulfat und engt sie im Vakuum zur Trockne ein. Der Rückstand wird aus Essigester umkristallisiert. Man erhält so 11,65 g [4.5-Bis-(4-methoxyphenyl)-2-imidazolyl]-(1.3-dioxolan-2-yl-methyl)-sulfid vom Schmelzpunkt 118—9°C.

Beispiel 28

Zu einer Lösung von 1,99 g [4.5-Bis-(4-methoxyphenyl)-2-imidazolyl]-1.3-dioxolan-2-yl-methyl)-sulfid in 150 ml Dichlormethan werden 1,19 g (80%) 3-Chlorperbenzoesäure in 100 ml Dichlormethan getropft. Man rührt 4 Stunden bei Raumtemperatur, wäscht dann mit Natriumbikarbonat-Lösung, trocknet die organische Lösung über Natriumsulfat und engt sie im Vakuum ein. Der Rückstand wird aus Essigester/Hexan kristallisiert. Nach Umkristallisation aus Essigester/Hexan erhält man 1,43 g [4.5-Bis-(4-methoxyphenyl)-2-imidazolyl]-(1.3-dioxolan-2-yl-methyl)-sulfoxid vom Schmelzpunkt 203—4°C.

Beispiel 29

Zu einer Lösung von 1,99 g [4.5-Bis-(4-methoxyphenyl)-2-imidazolyl]-(1.3-dioxolan-2-yl-methyl)-sulfid in 150 ml Dichlormethan wird die Lösung von 2,38 g (80%) 3-Chlorperbenzoesäure in 150 ml Dichlormethan getropft. Man rührt über Nacht bei Raumtemperatur, wäscht dann mit Natriumbikarbonat-Lösung, trocknet die organische Lösung über Natriumsulfat und engt sie im Vakuum ein. Der Rückstand wird aus Essigester/Äthanol kristallisiert. Nach Umkristallisation aus Essigester/Äthanol erhält man 1,71 g [4.5-Bis-(4-methoxyphenyl)-2-imidazolyl]-(1.3-dioxolan-2-yl-methyl)-sulfon vom Schmelzpunkt 150°C.

Beispiel 30

Zu einer Lösung von 320 mg Natrium in 100 ml absolutem Methanol werden unter Rühren 3,95 g 4.5-Bis-(4-methoxyphenyl)-2-mercaptoimidazol gegeben. Die klare, gelbe Lösung wird mit der Lösung von 2,53 g Bromacetaldehyddiäthylacetal in 30 ml absolutem Methanol versetzt. Man erhitzt 48 Stunden unter Argon am Rückfluß, läßt erkalten und gießt in 600 ml Eiswasser. Das Produkt wird mit Essigester extrahiert. Die organische Lösung wird über Natriumsulfat getrocknet und im Vakuum zur Trockne eingeengt. Durch Kristallisation aus Aceton/Hexan entfernt man unumgesetztes Imidazol. Die Mutterlauge wird dann in Hexan eingerührt. Das ausgefallene Öl wird abgetrennt und im Ölpumpenvakuum bei 50°C getrocknet. Man erhält so 2,93 g 4.5-Bis-(4-methoxyphenyl)-2-(2.2-diäthoxyäthylthio)-imidazol als gelbliches, viskoses Öl.

| | | | | | |
|---|---|---|---|---|---|
| $C_{23}H_{28}N_2O_4S$ (428.55) | Ber. | C 64,46 | H 6,58 | N 6,54 | S 7,48 |
| | Gef. | 64,49 | 6,69 | 6,48 | 7,22 |

Beispiel 31

Zu einer Lösung von 6,24 4.5-Bis-(4-methoxyphenyl)-2-mecaptoimidazol in 100 ml absolutem Dimethylformamid werden 0,96 g 50% Natriumhydrid gegeben, und es wird 30 Minuten auf 60°C erwärmt. Dann versetzt man mit einer Lösung von 4,33 g Bromacetaldehyddiäthylacetal in 20 ml absolutem Dimethylformamid und rührt 4 Stunden bei 80°C unter Argon. Man läßt abkühlen, gießt in 800 ml Eiswasser und extrahiert mit Chloroform. Die organische Lösung wird über Natriumsulfat getrocknet und im Vakuum zur Trockne eingeenigt. Der Rückstand wird, wie unter Beispiel 39 beschrieben, behandelt, und man erhält so 6,53 g 4.5-Bis-(4-methoxyphenyl)-2-(2.2-diäthoxyäthylthio)-imidazol als gelbes Öl.

| | | | | | |
|---|---|---|---|---|---|
| $C_{23}H_{28}N_2O_4S$ (428.55) | Ber. | C 64,46 | H 6,58 | N 6,54 | S 7,48 |
| | Gef. | 64,53 | 6,68 | 6,57 | 7,31 |

Beispiel 32

Zu einer Lösung von 4,29 g 4.5-Bis-(4-methoxyphenyl)-2-(2.2-diäthoxyäthylthio)-imidazol in 150 ml Methylenchlorid werden bei Raumtemperatur 2,38 g (80%) 3-Chlorperbenzoesäure in 150 ml Methylenchlorid getropft. Es wird 2 Stunden bei Raumtemperatur gerührt und mit $NaHCO_3$-Lösung gewaschen. Die organische Lösung wird über $Na_2SO_4$ getrocknet und im Vakuum eingeengt. Das verleibende Öl wird in Diäthyläther aufgenommen und durch Zugabe von Diisopropyläther zur Kristallisation gebracht. Man erhält so 3,49 g 4.5-Bis-(4-methoxyphenyl)-2-(2.2-diäthoxyäthylsulfinyl)-imidazol vom Schmelzpunkt 173—74°C.

Beispiel 33

Zu einer Lösung von 4,29 g 4.5-Bis-(4-methoxyphenyl)-2-(2.2-diäthoxyäthylthio)-imidazol in 150 ml Methylenchlorid werden bei Raumtemperatur 4,76 g (80%) 3-Chlorperbenzoesäure in 150 ml Methylenchlorid getropft. Es wird 1,5 Stunden bei Raumtemperatur gerührt und mit $NaHCO_3$-Lösung gewaschen. Die organische Lösung wird über $Na_2SO_4$ getrocknet und im Vakuum eingeengt. Das verbliebende Öl wird, wie unter Beispiel 40 beschrieben, behandelt, und man erhält so 3,09 g 4.5-Bis-(4-methoxyphenyl)-2-(2.2-diäthoxyäthyl-sulfonyl)-imidazol vom Schmelzpunkt 149—50°C.

Beispiel 34

Zu einer Lösung von 3,95 g 4.5-Bis-(4-methoxyphenyl)-2-mercaptoimidazol in 100 ml absolutem Methanol, in dem 320 mg Natrium gelöst sind, werden 2,53 g 3-Chlorpropanol gegeben, und man erhitzt 24 Stunden unter Argon am Rückfluß. Die erkaltete Lösung wird in 500 ml Wasser gegossen, mit 2 n Schwefelsäure neutralisiert und mit Chloroform extrahiert. Man trocknet die organische Lösung über Natriumsulfat, engt sie im Vakuum zur Trockne ein und kristallisiert den Rückstand aus Äthanol. Man erhält so 2,98 g 4.5-Bis-(4-methoxyphenyl)-2-(3-hydroxypropylthio)-imidazol vom Schmelzpunkt 163° C.

Beispiele 35

Zu einer Lösung von 506 mg Natrium in 150 ml absolutem Äthanol werden unter Rühren 6,24 g 4.5-Bis-(4-methoxyphenyl)-2-mercaptoimidazol gegeben. Die klare, gelbe Lösung wird dann mit der Lösung von 1,24 g Propylenoxid in 40 ml absolutem Äthanol versetzt. Man rührt 2 Stunden unter Argon bei Raumtemperatur, gießt dann in 600 ml Eiswasser, saugt die ausgefallenen Kristalle ab, wäscht sie mit Wasser und trocknet sie im Vakuum bei 70°C. Man erhält so 5,89 g 4.5-Bis-(4-methoxyphenyl)-2-(2-hydroxypropylthio)-imidazol vom Schmelzpunkt 163°C.

Beispiel 36

Zu einer Lösung von 483 mg Natrium in 100 ml absolutem Äthanol werden unter Rühren 6,24 g 4.5-Bis(4-methoxyphenyl)-2-mercaptoimidazol gegeben. Die klare, gelbe Lösung wird dann mit der Lösung von 1,59 g Isobutenoxid in 30 ml absolutem Äthanol versetzt, und man rührt 3 Stunden unter Argon bei Raumtemperatur. Dann gießt man in 600 ml Eiswasser, saugt die ausgefallenen Kristalle ab, wäscht sie mit Wasser und trocknet sie im Vakuum bei 70°C. Nach Umkristallisation aus Benzol erhält man 6,57 g 4.5-Bis-(4-methoxyphenyl)-2-(2-hydroxy-2-methyl-propylthio)-imidazol vom Schmelzpunkt 185°C.

Beispiel 37

Ein Gemisch aus 7,81 g 4.5-Bis-(4-methoxyphenyl)-2-mercaptoimidazol und 4,87 g 1-Bromäthylmethyläther wird in 150 ml Äthanol 3 Stunden unter Argon am Rückfluß erhitzt. Man läßt erkalten, neutralisiert mit 2 n Natronlauge und gießt in 1200 ml Eiswasser. Die ausgefallenen Kristalle werden abgesaugt, mit Wasser gewaschen und im Vakuum bei 50°C getrocknet. Das Rohprodukt wird aus Essigester umkristallisiert, und man erhält 6,37 g 4.5-Bis-(4-methoxyphenyl)-2-(2-methoxyäthylthio)-imidazol vom Schmelzpunkt 139°C.

**Patentansprüche für die Vertragsstaaten: BE CH DE FR GB IT LU LI NL SE**

1. Imidazol-Derivate zur Behandlung entzündlicher oder allergischer Erkrankungen des Menschen, gekennzeichnet durch die allgemeinen Formel I

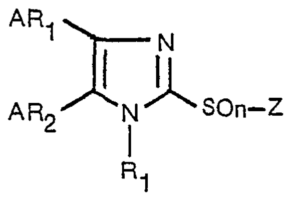

(I),

worin

n die Ziffern 0, 1 oder 2 darstellt, worin $AR_1$ und $AR_2$ einen durch Halogenatome, Alkylreste mit 1 bis 4 Kohlenstoffatomen oder Alkoxyresten mit 1 bis 4 Kohlenstoffatomen substituierter Phenylrest bedeutet, worin $R_1$ ein Wasserstoffatom oder eine Alkylgruppe mit 1 bis 4 Kohlenstoffatomen darstellt und worin

Z eine durch ein oder zwei Hydroxygruppen, Alkoxygruppen mit 1 bis 4 Kohlenstoffatomen, oder Alkylendioxygruppen mit 2 bis 6 Kohlenstoffatomen substituierter Alkylrest mit 2 bis 6 Kohlenstoffatomen bedeutet und deren Salze mit physiologisch unbedenklichen Säuren.

2. Imidazol-Derivate zur Behandlung entzündlicher oder allergischer Erkrankungen des Menschen der allgemeinen Formel I gemäß Patentanspruch 1, dadurch gekennzeichnet, daß die Substituenten $AR_1$ und $AR_2$ eine 4-Fluorphenylrest, einen 4-Chlorphenylrest, einen 4-Methylphenylrest oder einen 4-Methoxyphenylrest darstellen.

3. Imidazol-Derivate zur Behandlung entzündlicher oder allergischer Erkrankungen des Menschen der allgemeinen Formel I gemäß Patentanspruch 1 und 2, dadurch gekennzeichnet, daß der Substituent $R_1$ ein Wasserstoffatom bedeutet.

4. Imidazol-Derivate zur Behandlung entzündlicher oder allergischer Erkrankungen des Menschen der allgemeinen Formel I gemäß Patentanspruch 1 bis 3, dadurch gekennzeichnet, daß n die Ziffern 1 oder 2 bedeutet.

5. Imidazol-Derivate zur Behandlung entzündlicher oder allergischer Erkrankungen des Menschen der allgemeinen Formel I gemäß Patentanspruch 1 bis 4, dadurch gekennzeichnet, daß der Substituent Z eine Hydroxyalkylgruppe mit 2 bis 6 Kohlenstoffatomen bedeutet.

6. Imidazol-Derivate zur Behandlung entzündlicher oder allergischer Erkrankungen des Menschen der allgemeinen Formel I gemäß Patentanspruch 5, dadurch gekennzeichnet, daß der Substituent Z eine 2-Hydroxyäthylgruppe darstellt.

7. 4,5-Bis-(4-methoxyphenyl)-2-(2-hydroxyäthylthio)-imidazol zur Behandlung entzündlischer oder allergischer Erkrankungen des Menschen.

8. Imidazol-Derivate zur Behandlung entzündlicher oder allergischer Erkrankungen des Menschen der allgemeinen Formel I gemäß Patentanspruch 1 bis 4, dadurch gekennzeichnet, daß der Substituent Z eine 1,3-Dioxolan-2-ylmethylgruppe oder eine 2,2-Diäthoxyäthylgruppe bedeutet.

9. 4,5-Bis-(4-methoxyphenyl)-2-(2-hydroxyäthylsulfinyl)-imidazol.

10. 4,5-Bis-(4-methoxyphenyl)-2-(2-hydroxyäthylsulfonyl)-imidazol.

11. 4,5-Bis-(4-methoxyphenyl)-2-(hydroxyäthylthio)-1-methylimidazol.

12. 4,5-Bis-(4-fluorphenyl)-2-(2-hydroxyäthylthio)-imidazol.

13. 4,5-Bis-(4-fluorphenyl)-2-(2-hydroxyäthylsulfinyl)-imidazol.

14. 4,5-Bis-(4-fluorphenyl)-2-(2-hydroxyäthylsulfonyl)-imidazol.

15. 4,5-Bis-(4-chlorphenyl)-2-(2-hydroxyäthylthio)-imidazol zur Behandlung entzündlicher oder allergischer Erkrankungen des Menschen.

16. 4,5-Bis-(4-chlorphenyl)-2-(2-hydroxyäthylsulfinyl)-imidazol.

17. 4,5-Bis-(p-tolyl)-2-(2-hydroxyäthylthio)-imidazol.

18. 4,5-Bis-(p-tolyl)-2-(2-hydroxyäthylsulfinyl)-imidazol.

19. 4,5-Bis-(p-tolyl)-2-(2-hydroxyäthylsulfonyl)-imidazol.

20. 4,5-Bis-(4-methoxyphenyl)-2-(2-hydroxy-1-methyl-äthylthio)-imidazol.

21. 4,5-Bis-(4-methoxyphenyl)-2-(2-hydroxy-1-methyl-äthylsulfinyl)imidazol.

22. 4,5-Bis-(4-methoxyphenyl)-2-(2-hydroxy-1-methyl-äthylsulfonyl)imidazol.

23. 4,5-Bis-(4-methoxyphenyl)-2-(1,1-dimethyl-2-hydroxyäthylthio)-imidazol.

24. 4,5-Bis-(4-methoxyphenyl)-2-(1,1-dimethyl-2-hydroxyäthylsulfinyl)-imidazol.

25. 4,5-Bis-(4-methoxyphenyl)-2-(1,1-dimethyl-2-hydroxyäthylsulfonyl)-imidazol.

26. [4,5-Bis-(4-methoxyphenyl)-2-imidazolyl]-2-thio-1,4-butandiol.
27. 4,5-Bis-(4-methoxyphenyl)-2-(2,2-dimethoxyäthylthio)-imidazol.
28. 4,5-Bis-(4-methoxyphenyl)-2-(2,2-dimethoxyäthylsulfinyl)-imidazol.
29. 4,5-Bis-(4-methoxyphenyl)-2-(2,2-dimethoxyäthylsulfonyl)-imidazol.
30. [4,5-Bis-(4-methoxyphenyl)-2-imidazolyl]-(1,3-dioxolan-2-yl)-methyl)-sulfid.
31. [4,5-Bis-(4-methoxyphenyl)-2-imidazolyl]-(1,3-dioxolan-2-ylmethyl)-sulfoxid.
32. [4,5-Bis-(4-methoxyphenyl)-2-imidazolyl]-(1,3-dioxolan-2-ylmethyl)-sulfon.
33. 4,5-Bis-(4-methoxyphenyl)-2-(2,2-diäthoxyäthylthio)-imidazol.
34. 4,5-Bis-(4-methoxyphenyl)-2-(2,2-diäthoxyäthylsulfinyl)-imidazol.
35. 4,5-Bis-(4-methoxyphenyl)-2-(2,2-diäthoxyäthylsulfonyl)-imidazol.
36. 4,5-Bis-(4-methoxyphenyl)-2-(2-hydroxypropylthio)-imidazol.
37. 4,5-Bis-(4-methoxyphenyl)-2-(2-hydroxy-2-methylpropylthio)-imidazol.
38. Pharmazeutische Präparate zur Behandlung entzündlicher oder allergischer Erkrankungen des Menschen, gekennzeichnet durch ein oder zwei Imidazol-Derivate gemäß Anspruch 1 bis 37 in Kombination mit den in der galenischen Pharmazie üblichen Trägermitteln.

**Patentanspruch für den Vertragsstaat: AT**

1. Verfahren zur Herstellung von Indol-Derivaten der allgemeinen Formel I

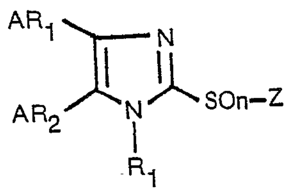

(I),

worin

n die Ziffern 0, 1 oder 2 darstellt, worin $AR_1$ und $AR_2$ einen durch Halogenatome, Alkylreste mit 1 bis 4 Kohlenstoffatomen oder Alkoxyresten mit 1 bis 4 Kohlenstoffatomen substituierter Phenylrest bedeutet, worin $R_1$ ein Wasserstoffatom oder eine Alkylgruppe mit 1 bis 4 Kohlenstoffatomen darstellt und worin.

Z eine durch ein oder zwei Hydroxygruppen, Alkoxygruppen mit 1 bis 4 Kohlenstoffatomen, oder Alkylendioxygruppen mit 2 bis 6 Kohlenstoffatomen substituierter Alkylrest mit 2 bis 6 Kohlenstoffatomen bedeutet und deren Salze mit physiologisch unbedenklichen Säuren dadurch gekennzeichnet, daß man in an sich bekannter Weise.

a) ein Imidazol-Derivat der allgemeinen Formel II

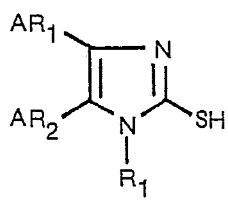

(II),

worin $AR_1$, $AR_2$ und $R_1$ die obengenannte Bedeutung besitzt, mit einer Verbindung der allgemeinen Formel III

$$W—Z \qquad (III),$$

worin Z die obengenannter Bedeutung besitzt und worin W ein Halogenatom, einen Alkylsulfonyloxyrest oder einen Arylsulfonyloxyrest darstellt, kondensiert, oder

b) zur Herstellung von Imidazol-Derivaten der allgemeinen Formel I mit Z in der Bedeutung einer Gruppierung

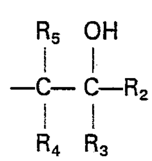

worin $R_2$, $R_3$, $R_4$ und $R_5$ Wasserstoffatome oder gegebenenfalls durch eine Hydroxygruppe, Alkoxygruppe oder Alkylendioxygruppe substituierte Kohlenwasserstoffreste mit insgesamt 1 bis 4 Kohlenstoffatomen bedeuten, an ein Imidazol-Derivat der allgemeinen Formel II

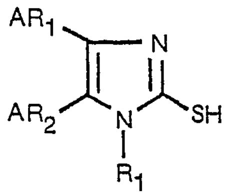

(II),

worin $AR_1$, $AR_2$ und $R_1$ die obengenannte Bedeutung besitzen, ein Epoxid der allgemeinen Formel IV

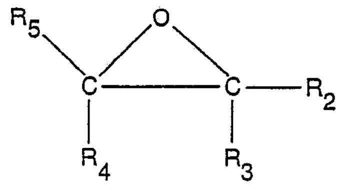

worin $R_2$, $R_3$, $R_4$ und $R_5$ die obengenannte Bedeutung besitzen addiert und gegebenenfalls die gemäß Verfahrensvariante a oder b erhaltenen Thioverbindungen der allgemeinen Formel I zu den entsprechenden Sulfinylverbindungen oder Sulfonylverbindungen oxydiert, gegebenenfalls in 1-Stellung unsubstituierte Imidazol-Derivate alkyliert und/oder Imidazol-Derivate der allgemeinen Formel I mit physiologisch unbedenklichen Säuren in ihre Salze überführt.

**Revendications pour les Etats contractants: BE CH DE FR GB IT LI LU NL SE**

1. Composés imidazoliques pour le traitement de maladies inflammatoires ou allergiques de l'homme, composés caractérisés en ce qu'ils répondent à la formule générale I:

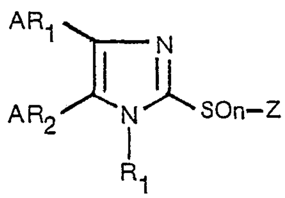

(I),

dans laquelle

n représente un nombre égal à 0, à 1 ou à 2,

$AR_1$ et $AR_2$ représentent chacun un radical phényle porteur d'atomes d'halogènes, d'alkyles contenant de 1 à 4 atomes de carbone ou d'alcoxy contenant de 1 à 4 atomes de carbone,

$R_1$ représente un atome d'hydrogène ou un radical alkyle contenant de 1 à 4 atomes de carbone et

Z représente un radical alkyle qui contient de 2 à 6 atomes de carbone et qui porte un ou deux radicaux hydroxy, un ou deux radicaux alcoxy contenant chacun de 1 à 4 atomes de carbone, ou un radical alkylènedioxy contenant de 2 à 6 atomes de carbone, ainsi que les sels qu'ils forment avec des acides acceptables du point de vue physiologique.

2. Composés imidazoliques de formule générale I selon la revendication 1, utilisables pour le traitement de maladies inflammatoires ou allergiques de l'homme, composés caractérisés en ce que les substituants $AR_1$ et $AR_2$ représentent chacun un radical fluoro-4 phényle, chloro-4 phényle, méthyl-4 phényle ou méthoxy-4 phényle.

3. Composés imidazoliques de formule générale I selon l'une des revendications 1 et 2, utilisables pour le traitement de maladies inflammatoires ou allergiques de l'homme, composés caractérisés en ce que le symbole $R_1$ représente un atome d'hydrogène.

4. Composés imidazoliques de formule générale I selon l'une quelconque des revendications 1 à 3, utilisables pour le traitement de maladies inflammatoires ou allergiques de l'homme, composés caractérisés en ce que n désigne le nombre 1 ou le nombre 2.

5. Composés imidazoliques de formule générale I selon l'une quelconque des revendications 1 à 4, utilisables pour le traitement des maladies inflammatoires ou allergiques de l'homme, composés caractérisés en ce que le substituant Z est un radical hydroxyalkyle contenant de 2 à 6 atomes de carbone.

6. Composés imidazoliques de formule générale I selon la revendication 5, utilisables pour le traitement de maladies inflammatoires ou allergiques de l'homme, composés caractérisé en ce que le substituant Z est un radical hydroxy-2 éthyle.

7. Bis-(méthoxy-4 phényl)-4,5 (hydroxy-2 éthylthio)-2 imidazole pour le traitement de maladies inflammatoires ou allergiques de l'homme.

8. Composés imidazoliques de formule générale I selon l'une quelconque des revendications 1 à 4, utilisables pour le traitement de maladies inflammatoires ou allergiques de l'homme, composés

caractérisés en ce que le substituant Z est un radical (dioxolanne-1,3 yl-2)-méthyle ou un radical diéthoxy-2,2 éthyle.

9. Bis-(méthoxy-4 phényl)-4,5 (hydroxy-2 éthylsulfinyl)-2 imidazole.

10. Bis-(méthoxy-4 phényl)-4,5 (hydroxy-2 éthylsulfonyl)-2 imidazole.

11. Bis-(méthoxy-4 phényl)-4,5 (hydroxy-2 éthylthio)-2 méthyl-1 imidazole.

12. Bis-(fluoro-4 phényl)-4,5 (hydroxy-2 éthylthio)-2 imidazole.

13. Bis-(fluoro-4 phényl)-4,5 (hydroxy-2 éthylsulfinyl)-2 imidazole.

14. Bis-(fluoro-4 phényl)-4,5 (hydroxy-2 éthylsulfonyl)-2 imidazole.

15. Bis-(chloro-4 phényl)-4,5 (hydroxy-2 éthylthio)-2 imidazole pour le traitement de maladies inflammatoires ou allergiques de l'homme.

16. Bis-(chloro-4 phényl)-4,5 (hydroxy-2 éthylsulfinyl)-2 imidazole.

17. Bis-(p-tolyl)-4,5 (hydroxy-2 éthylthio)-2 imidazole.

18. Bis-(p-tolyl)-4,5 (hydroxy-2 éthylsulfinyl)-2 imidazole.

19. Bis-(p-tolyl)-4,5 (hydroxy-2 éthylsulfonyl)-2 imidazole.

20. [Bis-(méthoxy-4 phényl)-4,5 imidazolyl-2 thio]-2 propionate d'éthyle.

21. Bis-(méthoxy-4 phényl)-4,5 hydroxy-2 méthyl-1 éthylsulfinyl)-2 imidazole.

22. Bis-(méthoxy-4 phényl)-4,5 (hydroxy-2 méthyl-1 éthylsulfonyl)-2 imidazole.

23. Bis-(méthoxy-4 phényl)-4,5 (diméthyl-1,1 hydroxy-2 éthylthio)-2 imidazole.

24. Bis-(méthoxy-4 phényl)-4,5 (diméthyl-1,1 hydroxy-2 éthylsulfinyl)-2 imidazole.

25. Bis-(méthoxy-4 phényl)-4,5 (diméthyl-1,1 hydroxy-2 éthylsulfonyl)-2 imidazole.

26. [Bis-(méthoxy-4 phényl)-4,5 imidazolyl-2 thio]-2 butane-diol-1,4.

27. Bis-(méthoxy-4 phényl)-4,5 (diméthoxy-2,2 éthylthio)-2 imidazole.

28. Bis-(méthoxy-4 phényl)-4,5 (diméthoxy-2,2 éthylsulfinyl)-2 imidazole.

29. Bis-(butoxy-4 phényl)-4,5 (diméthoxy-2,2 méthylsulfonyl)-2 imidazole.

30. Sulfure de bis-(méthoxy-4 phényl)-4,5 imidazolyle-2 et (dioxolanne-1,3 yl-2)-méthyle.

31. Bis-(méthoxy-4 phényl)-4,5 (dioxolanne-1,3 yl-2 méthyl-sulfinyl)-2 imidazole.

32. Bis-(méthoxy-4 phényl)-4,5 (dioxolanne-1,3 yl-2 méthyl-sulfonyl)-2 imidazole.

33. Bis-(méthoxy-4 phényl)-4,5 (diéthoxy-2,2 éthylthio)-2 imidazole.

34. Bis-(méthoxy-4 phényl)-4,5 (diéthoxy-2,2 éthylsulfinyl)-2 imidazole.

35. Bis-(méthoxy-4 phényl)-4,5 (diethoxy-2,2 ethylsulfonyl-2 imidazole.

36. Bis-(méthoxy-4 phényl)-4,5 (hydroxy-2 propylthio)-2 imidazole.

37. Bis-(méthoxy-4 phényl)-4,5 (hydroxy-2 méthyl-2 propylthio)-2 imidazole.

38. Compositions pharmaceutiques pour le traitement de maladies inflammatoires ou allergiques de l'homme, compositions caractérisées en ce qu'elles contiennent un ou deux composés imidazoliques selon l'une quelconque des revendications 1 à 37, associées à des adjuvants pris parmi ceux dont on se sert habituellement en pharmacie.

**Revendication pour l'Etat Contractant: AT**

1. Procédé de préparation de composés indoliques répondant à la formule générale I:

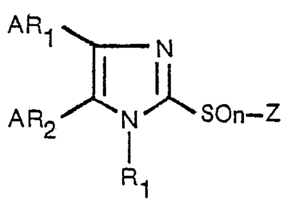

(I),

dans laquelle

n représente un nombre égal à 0, à 1 ou à 2,

$AR_1$ et $AR_2$ représentent chacun un radical phényle porteur d'atomes d'halogènes, d'alkyles contenant de 1 à 4 atomes de carbone ou d'alcoxy contenant de 1 à 4 atomes de carbone,

$R_1$ représente un atome d'hydrogène ou un radical alkyle contenant de 1 à 4 atomes de carbone et

Z représente un radical alkyl qui contient de 2 à 6 atomes de carbone et qui porte un ou deux radicaux hydroxy, un ou deux radicaux alcoxy contenant chacun de 1 à 4 atomes de carbone, ou un radical alkylène-dioxy contenant de 2 à 6 atomes de carbone, et des sels qu'ils forment avec des acides acceptables du point de vue biologique, procédé caractérisé en ce que, en opérant de manière connue:

a) On condense un dérivé de l'imidazole répondant à la formule générale II:

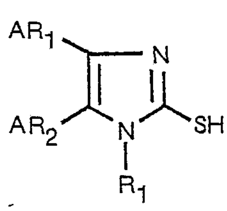

(II),

dans laquelle $AR_1$, $AR_2$ et $R_1$ ont les significations précédemment données, avec un composé répondant à la formule générale III:

$$W—Z \qquad (III)$$

dans laquelle Z a la signification précédemment donnée et W représente un atome d'halogène, un radical alkylsulfonyloxy ou un radical arylsulfonyloxy, ou

b) Pour préparer des dérivés de l'imidazole de formule générale I dans lesquelles Z désigne un groupement:

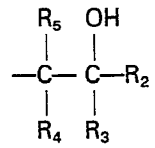

dans laquelle $R_2$, $R_3$, $R_4$ et $R_5$ représentent chacun un atome d'hydrogène ou un radical hydrocarboné qui porte éventuellement un radical hydroxy, alcoxy ou alkylène-dioxy et qui contient au total de 1 à 4 atomes de carbone, on fixe un époxyde répondant à la formule IV:

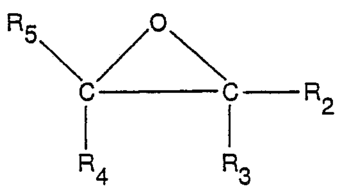

dans laquelle $AR_1$, $AR_2$ et $R_1$ ont les significations précédemment données, et éventuellement on oxyde les composés thio de formule générale I qui ont été obtenus selon l'une ou l'autre des variantes a) et b) de manière à les convertir en les composés sulfinyliques ou sulfonyliques correspondants, on alkyle éventuellement des dérivés de l'imidazole non substitués en position 1 et/ou on transforme des dérivés de l'imidazole de formule générale I en leurs sels avec des acides acceptables du point de vue physiologique.

**Claims for the Contracting States: BE CH DE FR GB IT LI LU NL SE**

1. Imidazole derivatives for the treatment of inflammatory or allergic diseases of humans characterised in that they are of general formula I

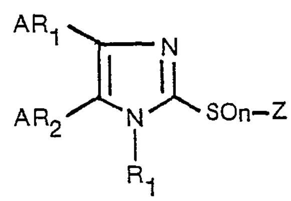

(I),

wherein

n is the integer 0, 1 or 2,

$AR_1$ and $AR_2$ are phenyl groups substituted by halogen, alkyl of 1 to 4 carbon atoms or alkoxy of 1 to 4 carbon atoms and

Z is an alkyl group of 2 to 6 carbon atoms substituted by one or two hydroxy groups, alkoxy groups of 1 to 4 carbon atoms or alkylenedioxy groups of 2 to 6 carbon atoms, and salts of these with physiologically acceptable acids.

2. Imidazole derivatives for the treatment of inflammatory or allergic diseases of humans of general formula I, characterized in that the substituents $AR_1$ and $AR_2$ are 4-fluorophenyl, 4-chlorophenyl, 4-methylphenyl or 4-methoxyphenyl.

3. Imidazole derivatives for the treatment of inflammatory or allergic disease of humans of general formula I, according to claims 1 and 2, characterised in that the substituent $R_1$ is hydrogen.

4. Imidazole derivatives for the treatment of inflammatory or allergic diseases of humans of general formula I, according to claims 1 to 3 characterised in that n is the integer 1 or 2.

5. Imidazole derivatives for the treatment of inflammatory or allergic diseases of humans of general formula I, according to claims 1 to 4 characterised in that the substituent Z is a hydroxyalkyl group of 2 to 6 carbon atoms.

6. Imidazole derivatives for the treatment of inflammatory or allergic diseases of humans of general formula I, according to claim 5 characterised in that the substituent Z is a 2-hydroxyethyl group.

7. 4,5-Bis-(4-methoxyphenyl)-2-(2-hydroxyethylthio)imidazole for the treatment of inflammatory or allergic diseases of humans.

8. Imidazole derivatives for the treatment of inflammatory or allergic diseases of humans of general formula I, according to claims 1 to 4 characterised in that the substituent Z is a 1,3-dioxolan-2-ylmethyl or a 2,2-diethoxyethyl group.

9. 4,5-Bis-(4-methoxyphenyl)-2-(2-hydroxyethylsulphinyl)-imidazole.

10. 4,5-Bis-(4-methoxyphenyl)-2-(2-hydroxyethylsulphonyl)-imidazole.

11. 4,5-Bis-(4-methoxyphenyl)-2-(2-hydroxyethylthio)-1-methylimidazole.

12. 4,5-Bis-(4-fluorophenyl)-2-(2-hydroxyethylthio)-imidazole.

13. 4,5-Bis-(4-fluorophenyl)-2-(2-hydroxyethylsulphinyl)-imidazole.

14. 4,5-Bis-(4-fluorophenyl)-2-(2-hydroxyethylsulphonyl)-imidazole.

15. 4,5-Bis-(4-chlorophenyl)-2-(2-hydroxyethylthio)-imidazole for the treatment of inflammatory or allergic diseases of humans.

16. 4,5-Bis-(4-chlorophenyl)-2-(2-hydroxyethylsulphinyl)-imidazole.

17. 4,5-Bis-(p-tolyl)-2-(2-hydroxyethylthio)-imidazole.

18. 4,5-Bis-(p-tolyl)-2-(2-hydroxyethylsulphinyl)-imidazole.

19. 4,5-Bis-(p-tolyl)-2-(2-hydroxyethylsulphonyl)-imidazole.

20. 4,5-Bis-(4-methoxyphenyl)-2-(2-hydroxy-1-methylethylthio)-imidazole.

21. 4,5-Bis-(4-methoxyphenyl)-2-(2-hydroxy-1-methylethylsulphinyl)-imidazole.

22. 4,5-Bis-(4-methoxyphenyl)-2-(2-hydroxy-1-methylethylsulphonyl)-imidazole.

23. 4,5-Bis-(4-methoxyphenyl)-2-(1,1-dimethyl-2-hydroxyethylthio)-imidazole.

24. 4,5-Bis-(4-methoxyphenyl)-2-(1,1-dimethyl-2-hydroxyethylsulphinyl)-imidazole.

25. 4,5-Bis-(4-methoxyphenyl)-2-(1,1-dimethyl-2-hydroxyethylsulphonyl)-imidazole.

26. [4,5-Bis-(4-methoxyphenyl)-2-imidazolyl]-2-thio-1,4-butanediol.

27. 4,5-Bis-(4-methoxyphenyl)-2-(2,2-dimethoxyethylthio)-imidazole.

28. 4,5-Bis-(4-methoxyphenyl)-2-(2,2-dimethoxyethylsulphinyl)-imidazole.

29. 4,5-Bis-(4-methoxyphenyl)-2-(2,2-dimethoxyethylsulphonyl)-imidazole.

30. [4,5-Bis-(4-methoxyphenyl)-2-imidazolyl]-(1,3-dioxolan-2-ylmethyl)sulphide.

31. [4,5-Bis-(4-methoxyphenyl)-2-imidazolyl]-(1,3-dioxolan-2-ylmethyl)sulphoxide.

32. [4,5-Bis-(4-methoxyphenyl)-2-imidazolyl]-(1,3-dioxolan-2-ylmethyl)sulphone.

33. 4,5-Bis-(4-methoxyphenyl)-2-(2,2-diethoxyethylthio)-imidazole.

34. 4,5-Bis-(4-methoxyphenyl)-2-(2,2-diethoxyethylsulphinyl)-imidazole.

35. 4,5-Bis-(4-methoxyphenyl)-2-(2,2-diethoxyethylsulphonyl)-imidazole.

36. 4,5-Bis-(4-methoxyphenyl)-2-(2-hydroxypropylthio)-imidazole.

37. 4,5-Bis-(4-methoxyphenyl)-2-(2-hydroxy-2-methylpropylthio)-imidazole.

38. Pharmaceutical compositions for the treatment of inflammatory or allergic diseases of humans characterised in that they comprise one or two imidazole derivatives according to claims 1 to 37 in admixture with conventional pharmaceutical carriers.

**Claim for the Contracting State: AT**

1. Process for the preparation of indole derivatives of general formula I

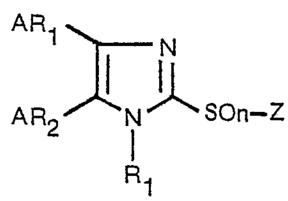

(I),

wherein

n is the integer 0, 1 or 2,

$AR_1$ and $AR_2$ are phenyl groups substituted by halogen, alkyl of 1 to 4 carbon atoms or alkoxy of 1 to 4 carbon atoms and

Z is an alkyl group of 2 to 6 carbon atoms substituted by one or two hydroxy groups, alkoxy groups of 1 to 4 carbon atoms and alkylendioxy groups of 2 to 6 carbon atoms, and salts of these with physiologically acceptable acids characterised in that in a known manner.

a) an imidazole derivative of general formula II

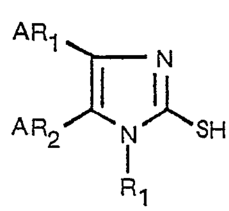

(II),

in which $AR_1$, $AR_2$ and $R_1$ have the meanings given above, is condensed with a compound of general formula III

W—Z III

in which Z has the meaning given above and W is halogen atom, an alkylsulphonyloxy group or an arylsulphonyloxy group, or

b) for the preparation of imidazole derivatives of general formula I where Z is a group

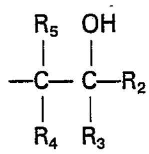

in which $R_2$, $R_3$, $R_4$ and $R_5$ are hydrogen, or hydrocarbyl groups of 1 to 4 carbon atoms, optionally substituted by hydroxy, alkoxy or alkylenedioxy, an epoxide of general formula IV

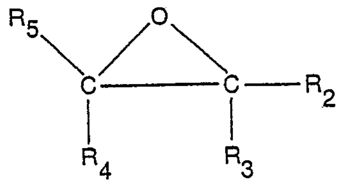

in which $R_2$, $R_3$, $R_4$ and $R_5$ have the above given meanings is added to an imidazole derivative of general formula II

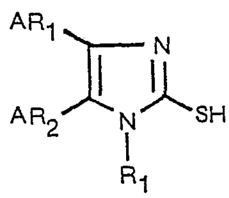

(II),

in which $AR_1$, $AR_2$ and $R_1$ have the above given meanings, and if necessary, the thio compounds of formula I, obtained by processes a or b are oxidised to the corresponding sulphinyl or sulphonyl compounds, if necessary an imidazole derivative unsubstituted in the 1-position is alkylated and/or imidazole derivatives of general formula I are converted into their salts with physiologically acceptable acids.